# EUROPEAN PATENT SPECIFICATION

(11) **EP 2 248 859 B1**
(45) Date of publication and mention of the grant of the patent: **10.07.2013**
(21) Application number: 08800705.9
(22) Date of filing: 29.08.2008
(51) Int. Cl.: C09B 62/01, C07C 245/22, C09B 62/443, C09B 62/453, D06P 3/14

(54) **A BLACK REACTIVE DYE AND THE PREPARATION THEREOF**
SCHWARZER REAKTIVFARBSTOFF UND SEIN HERSTELLUNGSVERFAHREN
COLORANT NOIR RÉACTIF ET LA PREPARATION DUDIT COMPOSE

(30) Priority: 22.01.2008 CN 200810052126
(43) Date of publication of application: 10.11.2010
(73) Proprietor: Tianjin Dek Chemical Co., Ltd, Tianjin 300163 (CN)
(72) Inventor: ZHANG, Kedong, Tianjin 300163 (CN); ZHANG, Xinghua, Tianjin 300163 (CN); XING, Guangwen, Tianjin 300163 (CN)
(74) Representative: Held, Stephan
(86) International application number: PCT/CN2008/072193
(87) International publication number: WO 2009/092210

(56) References cited:
- EP-A1- 0 071 035
- WO-A1-03/033599
- CN-A- 1 136 057
- CN-A- 1 136 057
- CN-A- 101 054 476
- CN-A- 101 215 427
- CN-A- 101 235 217
- US-A- 2 657 205
- US-A- 4 257 770
- XIAO JIN-QIU ET AL: "Study of levelling properties of SN type reactive dyes on wool fabric", DALIAN LIGONG DAXUE XUEBAO - JOURNAL OF DALIAN UNIVERSITY OFTECHNOLOGY, GAI KAN BIANJIBU, DALIAN, CN, vol. 43, no. 5, 1 January 2003 (2003-01-01), pages 586-590, XP009147039, ISSN: 1000-8608

## Description

### Filed of the invention

The present invention relates to a kind of reactive dyes and the preparation of the same, especially relates to black reactive dyes used in staining the fiber, such as wool, cotton, silk, polyamide and so on, and the preparation of the same.

### Background of the invention

Staining the woolen textiles usually use acid dyes, acid mordant dyes or metal complex dyes. The color fastness of acid dyes is mediocre, and it has damage to the wool fiber, so it can not be used for staining the worsted fabric. Although the mordant dyes and metal complex dyes have higher color fastness, it is dim, and when staining , the heavy metal Cr+ is exist, making the treatment of staining waste water difficult.

With the development of wool industry, the requirements for quality of woolen textiles is higher and higher, the comfort and security of woolen textiles have been to the new focus. The dark dyes especially the black dye is always the main product used in staining woolen textiles. Especially cashmere, the top grade of woolen textiles, has more strict requirements to the variety of black dyes, high fixation yield, good blackening , high color fastness, good dyeing uniformity, when staining the damage to fibers is little, having litter of staining wastewater and the COD is relatively low.

Resently, there is not a kind of reactive black dyes used in wool meet the requirement above. It only can use metal complex dyes-acid mordant black PV in black dyeing of woolen textiles. When staining the cashmere with the dyes, it has good blackening, high color fastness and high fixation yield, but it need add plenty of heavy metal Cr⁶⁺ when staining, it has destruction either to people or to the environment. It need add plenty of heavy metal Cr⁶⁺ when staining ,after staining, plenty of Cr⁶⁺ residue in waste liquor, it is difficult to abate and high cost, and it is difficult to realize complete control and discharge standards. Now it has been Internationally forbidden restrict and forbidden the kind of wool textiles dyeing by the dyes enter U.S. and European markets.

Some substitutes of acid mordant black PV have ever brought out home and abroad, but both the blackening and fixation yield can not realize the same level, and can't really replace the mordant black PV.

EP0071035 discloses reactive mono-functional **blue-black disazo** dyes.

### Brief summary of the invention

The present invention will solve the technical problems, which are provide a kind of black reactive dyes with good performance, high fixation yield and environment-friendly, and the preparation of the same.

In order to solve the problems above, technical scheme adopted by the present invention is :

A kind of black reactive dyes, represented by the formula 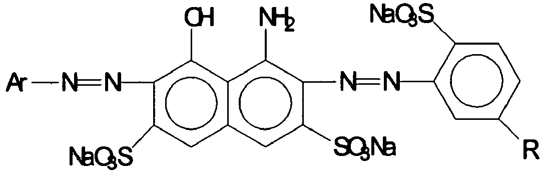 Where
R=-NHCOCHBrCH₂Br -NHCOCBrCH₂ or -SO₂CH=CH₂

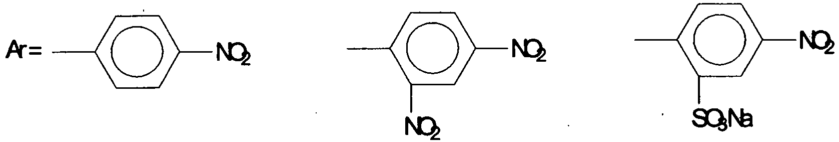

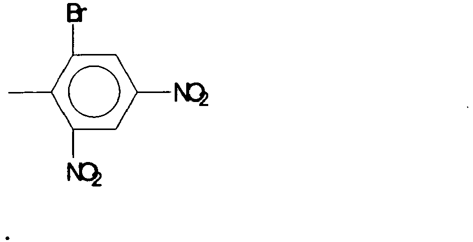

The method of preparing black reactive dyes, including the approaches as follows:
a. the preparation of condensate diazonium salt: 2,4-biaminobenzene sulfonic acid sodium was added to water, control the temperature at 2-8°C through using ice, 2,3-bibromopropionyl chloride was added, control the pH=6-8 through using 10-20% sodium carbonate solution, react for 3-6 hours, control the react temperature at 2-8°C,the suspension of condensate was obtained; hydrochloric acid and ice were added to the suspension of condensate, cool to 0-5°C,sodium nitrite was added, reacted at 7-14 °C for 1-2 hours, keeping slight excess of sodium nitrite in the process of reacting , when complete , amino sulfonic acid was added for removing the excess sodium nitrite;
b. the preparation of dinitroaniline diazonium salt: sodium nitrite was added to 98% sulfuric acid, control the temperature below 30°C, after adding, increased to 65-70°C, keep 2-3 hours; drop the temperature to 30°C below, dinitroaniline was added, control the temperature below 40°C, after adding ,keep 40±3°C for more than 6 hours, drop to 30°C below ,dilute to the mixture of ice and water, the temperature is 2°C below , amino sulfonic acid was added for removing the excess sodium nitrite;
c. the preparation of H acid solution: quantitative H acid was added to water, use 30-40% sodium hydroxide solution neutralize to pH=6-7, make it dissolve;
d. the first step coupling: sodium bicarbonate was added to the diazonium salt of approach a, adjust pH=1.5-2.5, add the H acid of approach , control the temperature at 10-15°C, react for more than 6 hours , obtain red intermediate;
e. the second step of coupling: diazonium salt react solution of approach b was added to the red intermediate of approach d, use the sodium carbonate to control the pH=5-7, react for 2-3 hours , rised to 30-35°C, add 10-20% of the whole volume sodium chloride or potassium chloride, stir and salt out for 2-4 hours ,filter, the filter cake is the semi-finished product of the black reactive dyes;
f. post treatment: beating and dissolving the semi-finished of approach e, the solution spray drying , obtain reactive black product, orange reactive dyes or red reactive dyes were added, the hight jet black reactive dyes was obtained.

The method of preparing black reactive dyes, including the approaches as follows:
a. the preparation of condensate diazonium salt: 2,4-biaminobenzene sulfonic acid sodium was added to water, control the temperature at 2-8°C through using ice, 2,3-bibromopropionyl chloride was added, control the pH=6-8 through using 10-20% sodium carbonate solution, react for 3-6 hours, control the react temperature at 2-8°C,obtain the suspension of condensate; hydrochloric acid and ice were added to the suspension of condensate, cool to 0-5°C,add sodium nitrite, react at 7-14 °C for 1-2 hours, keep slight excess of sodium nitrite in the process of reacting , when complete , amino sulfonic acid was added for removing the excess sodium nitrite;
b. the preparation of p-nitroaniline diazonium salt: travel milk-12 surfactant, p-nitroaniline, and hydrochloric acid were added to water, beating for more than 1 hours, ice was added to drop to 0-5°C, sodium nitrite solution was added, reacting at 0-5°C for 2-3 hours, when the react was finished, amino sulfonic acid was added for removing the excess nitrous acid;
c. the preparation of H acid solution: H acid was added to water, use 30-40% sodium hydroxide solution neutralize to pH=6-7, make it dissolve;
d. the first step coupling: sodium bicarbonate was added to the diazonium salt of approach a, adjusting pH=1.5-2.5, the H acid of approach c was added, controling the temperature at 10-15°C, reacting for more than 6 hours , red intermediate was obtained;
e. the second step of coupling: diazonium salt react solution of approach b was added to the red intermediate of approach d, using the sodium carbonate to control the pH=5-7, reacted for 2-3 hours , and rised to 30-35°C, 10-20% of the whole volume sodium chloride or potassium chloride was added, stirred and salted out for 2-4 hours ,filtered, the filter cake was the semi-finished product of the black active dyes;
f. post treatment: beating and dissolving the semi-finished of approach e, the solution spray drying , obtain reactive black product, orange reactive dyes or red reactive dyes were added, the hight jet black reactive dyes was obtained.

The method of preparing black reactive dyes, including the approaches as follows:
a. the preparation of condensate diazonium salt: 2,4-biaminobenzene sulfonic acid sodium was added to water, control the temperature at 2-8°C through using ice, 2,3-bibromopropionyl chloride was added, control the pH=6-8 through using 10-20% sodium carbonate solution, react for 3-6 hours, control the react temperature at 2-8°C,the suspension of condensate was obtained; hydrochloric acid and ice were added to the suspension of condensate, cool to 0-5°C,sodium nitrite was added, reacted at 7-14 °C for 1-2 hours, keeping slight excess of sodium nitrite in the process of reacting , when complete , amino sulfonic acid was added for removing the excess sodium nitrite;
b. the preparation of p-nitroaniline ortho sulfonic acid diazonium salt: p-nitroaniline ortho sulfonic acid and hydrochloric acid were added to water, adding ice to drop to 0-5°C, sodium nitrite solution was added, reacting at 0-5°C for 4-6 hours, when the react was finished, amino sulfonic acid was added for removing the excess nitrous acid;
c. the preparation of H acid solution: H acid was added to water, use 30-40% sodium hydroxide solution neutralize to pH=6-7, make it dissolve;;
d. the first step coupling: sodium bicarbonate was added to the diazonium salt of approach a, adjusting pH=1.5-2.5, the H acid of approach c was added, controling the temperature at 10-15°C, reacting for more than 6 hours , red intermediate was obtained;
e. the second step of coupling: diazonium salt react solution of approach b was added to the red intermediate of approach d, using the sodium carbonate to control the pH=5-7, reacted for 2-3 hours , and rised to 30-35°C, 10-20% of the whole volume sodium chloride or potassium chloride was added, stirred and salted out for 2-4 hours ,filtered, the filter cake was the semi-finished product of the black active dyes;
f. post treatment: beating and dissolving the semi-finished of approach e, the solution spray drying , obtain reactive black product, orange reactive dyes or red reactive dyes were added, the hight jet black reactive dyes was obtained.

The method of preparing black reactive dyes, including the approaches as follows:
a. the preparation of m-sulfonated ester diazonium salt: m-sulfonated ester and hydrochloric acid were added to water, adding ice to drop to 0-5°C, sodium nitrite solution was added, reacted at 0-5°C for 4-6 hours, when the react was finished, amino sulfonic acid was added for removing the excess nitrous acid;
b. the preparation of dinitroaniline diazonium salt: sodium nitrite was added to 98% sulfuric acid, control the temperature below 30°C, after adding, increased to 65-70°C, keep 2-3 hours; drop the temperature to 30°C below, dinitroaniline was added, control the temperature below 40°C, after adding ,keep 40±3°C for more than 6 hours, drop to 30°C below ,dilute to the mixture of ice and water, the temperature is 2°C below , amino sulfonic acid was added for removing the excess sodium nitrite;
c. the first step coupling: directly adding H acid to the diazonium salt of approach a, controlling the temperature at 10-15°C, reacting for more than 6 hours. red intermediate was obtained;
d. the second step of coupling: diazonium salt react solution of approach c was added to the red intermediate of approach d, using the sodium carbonate to control the pH=5-7, reacted for 2-3 hours , and rised to 30-35°C, 10-20% of the whole volume sodium chloride or potassium chloride was added, stirred and salted out for 2-4 hours ,filtered, the filter cake was the semi-finished product of the black active dyes;
e. post treatment: beating and dissolving the semi-finished of approach d, the solution spray drying , obtain reactive black product, orange reactive dyes or red reactive dyes were added, the hight jet black reactive dyes was obtained.

The method of preparing black reactive dyes, including the approaches as follows:
a. the preparation of m-sulfonated ester diazonium salt: m-sulfonated ester and hydrochloric acid were added to water, adding ice to drop to 0-5°C, sodium nitrite solution was added, reacted at 0-5°C for 4-6 hours, when the react was finished, amino sulfonic acid was added for removing the excess nitrous acid;
b. the preparation of p-nitroaniline diazonium salt: travel milk-12 surfactant, p-nitroaniline, and hydrochloric acid were addedto water, beating for more than 1 hours, ice was added to drop to 0-5°C, sodium nitrite solution was added, reacting at 0-5°C for 2-3 hours, when the react was finished, amino sulfonic acid was added for removing the excess nitrous acid;
c. the first step coupling: directly adding H acid to the diazonium salt of approach a, controlling the temperature at 10-15°C, reacting for more than 6 hours . red intermediate was obtained;
d. the second step of coupling: diazonium salt react solution of approach c was added to the red intermediate of approach d, using the sodium carbonate to control the pH=5-7, reacted for 2-3 hours , and rised to 30-35°C, 10-20% of the whole volume sodium chloride or potassium chloride was added, stirred and salted out for 2-4 hours ,filtered, the filter cake was the semi-finished product of the black active dyes;
e. post treatment: beating and dissolving the semi-finished of approach d, the solution spray drying , obtain reactive black product, orange reactive dyes or red reactive dyes were added, the hight jet black black reactive dyes was obtained.

The method of preparing black reactive dyes, including the approaches as follows:
a. the preparation of m-sulfonated ester diazonium salt: m-sulfonated ester and hydrochloric acid were added to water, adding ice to drop to 0-5°C, sodium nitrite solution was added, reacted at 0-5°C for 4-6 hours, when the react was finished, amino sulfonic acid was added for removing the excess nitrous acid;
b. the preparation of p-nitroaniline ortho sulfonic acid diazonium salt: p-nitroaniline ortho sulfonic acid and hydrochloric acid were added to water, adding ice to drop to 0-5°C, sodium nitrite solution was added, reacting at 0-5°C for 4-6 hours, when the react was finished, amino sulfonic acid was added for removing the excess nitrous acid;
c. the first step coupling: directly adding H acid to the diazonium salt of approach a, controlling the temperature at 10-15°C, reacting for more than 6 hours . red intermediate was obtained;
d. the second step of coupling: diazonium salt react solution of approach c was added to the red intermediate of approach d, using the sodium carbonate to control the pH=5-7, reacted for 2-3 hours , and rised to 30-35°C, 10-20% of the whole volume sodium chloride or potassium chloride was added, stirred and salted out for 2-4 hours ,filtered, the filter cake was the semi-finished product of the black reactive dyes;
e. post treatment: beating and dissolving the semi-finished of approach d, the solution spray drying , obtain active black product, orange reactive dyes or red reactive dyes were added, the hight jet black reactive dyes was obtained.

The beneficial effect of the present invention is the dyes of the present invention come up to the strict requirement of wool textile industry, when staining the cashmere as the substitute of acid mordant black PV, fastness is equal to the acid mordant black PV, and the fixation yield and jet black level is equal to or a little better than the acid mordant black PV, the most notably advantage is that it does not need add heavy metals. Because of the wool reactive group react with fiber and forming covalent bond, the fixation yield and fastness improve. The product of the present invention fills up the vacancy of such products at home and abroad. It dose not contain any components forbidden, and when staining, it dose not need add Cr⁺, it is environment friendly dye product. It dose not only meet a number of technical requirements of top grade wool-cashmere to dyes, but also completely solve the problem of the difficulty of the governance of dying wastewater, and at the same time solve the problem that the jet black of wool used reactive black product is low. The product can also used in staining the fiber, such as cotton, silk, polyamide and so on, and has good promence and fastness, it is a kind of black reactive dyes that has widely value in use.

### Detailed description of the invention

The black reactive dyes of the present invention, represented by the formula 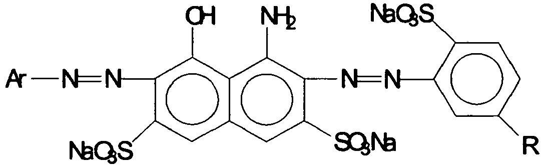 Where
R=-NHCOCHBrCH₂Br -NHCOCBrCH₂ or -SO₂CH=CH₂

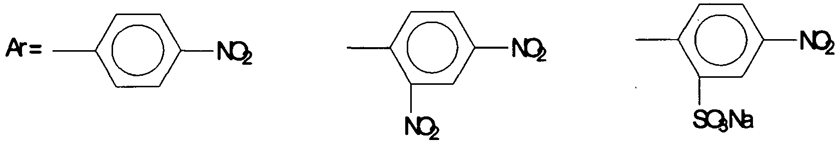

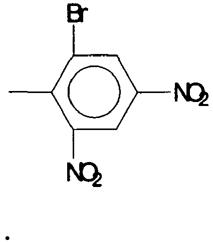

The method of preparing black reactive dyes, including the approaches as follows:
a. the preparation of condensate diazonium salt: 2,4-biaminobenzene sulfonic acid sodium was added to water, control the temperature at 2-8°C through using ice, 2,3-bibromopropionyl chloride was added, control the pH=6-8 through using 10-20% sodium carbonate solution, react for 3-6 hours, control the react temperature at 2-8°C,the suspension of condensate was obtained; hydrochloric acid and ice were added to the suspension of condensate, cool to 0-5°C,sodium nitrite was added, reacted at 7-14 °C for 1-2 hours, keeping slight excess of sodium nitrite in the process of reacting , when complete , amino sulfonic acid was added for removing the excess sodium nitrite;
b. the preparation of dinitroaniline diazonium salt: sodium nitrite was added to 98% sulfuric acid, control the temperature below 30°C, after adding, increased to 65-70°C, keep 2-3 hours; drop the temperature to 30°C below, dinitroaniline was added, control the temperature below 40°C, after adding ,keep 40+3°C for more than 6 hours, drop to 30°C below ,dilute to the mixture of ice and water, the temperature is 2°C below , amino sulfonic acid was added for removing the excess sodium nitrite;
c. the preparation of H acid solution: quantitative H acid was added to water, use 30-40% sodium hydroxide solution neutralize to pH=6-7, make it dissolve;
d. the first step coupling: sodium bicarbonate was added to the diazonium salt of approach a, adjust pH=1.5-2.5, add the H acid of approach , control the temperature at 10-15°C, react for more than 6 hours , obtain red intermediate;
e. the second step of coupling: diazonium salt react solution of approach b was added to the red intermediate of approach d, use the sodium carbonate to control the pH=5-7, react for 2-3 hours , rised to 30-35°C, add 10-20% of the whole volume sodium chloride or potassium chloride, stir and salt out for 2-4 hours ,filter, the filter cake is the semi-finished product of the black active dyes;
f. post treatment: beating and dissolving the semi-finished of approach e, the solution spray drying , obtain reactive black product, orange reactive dyes or red reactive dyes were added, the hight jet black reactive active dyes was obtained.

The method of preparing black reactive dyes, including the approaches as follows:
a. the preparation of condensate diazonium salt: 2,4-biaminobenzene sulfonic acid sodium was added to water, control the temperature at 2-8°C through using ice, 2,3-bibromopropionyl chloride was added, control the pH=6-8 through using 10-20% sodium carbonate solution, react for 3-6 hours, control the react temperature at 2-8°C,obtain the suspension of condensate; hydrochloric acid and ice were added to the suspension of condensate, cool to 0-5°C,add sodium nitrite, react at 7-14 °C for 1-2 hours, keep slight excess of sodium nitrite in the process of reacting , when complete , amino sulfonic acid was added for removing the excess sodium nitrite;
b. the preparation of p-nitroaniline diazonium salt: travel milk-12 surfactant, p-nitroaniline, and hydrochloric acid were added to water, beating for more than 1 hours, ice was added to drop to 0-5°C, sodium nitrite solution was added, reacting at 0-5°C for 2-3 hours, when the react was finished, amino sulfonic acid was added for removing the excess nitrous acid;
c. the preparation of H acid solution: H acid was added to water, use 30-40% sodium hydroxide solution neutralize to pH=6-7, make it dissolve;
d. the first step coupling: sodium bicarbonate was added to the diazonium salt of approach a, adjusting pH=1.5-2.5, the H acid of approach c was added, controling the temperature at 10-15°C, reacting for more than 6 hours , red intermediate was obtained;
e. the second step of coupling: diazonium salt react solution of approach b was added to the red intermediate of approach d, using the sodium carbonate to control the pH=5-7, reacted for 2-3 hours , and rised to 30-35°C, 10-20% of the whole volume sodium chloride or potassium chloride was added, stirred and salted out for 2-4 hours ,filtered, the filter cake was the semi-finished product of the black active dyes;
f. post treatment: beating and dissolving the semi-finished of approach e, the solution spray drying , obtain reactive black product, orange reactive dyes or red reactive dyes were added, the hight jet black reactive dyes was obtained.

The method of preparing black reactive dyes, including the approaches as follows:
a. the preparation of condensate diazonium salt: 2,4-biaminobenzene sulfonic acid sodium was added to water, control the temperature at 2-8°C through using ice, 2,3-bibromopropionyl chloride was added, control the pH=6-8 through using 10-20% sodium carbonate solution, react for 3-6 hours, control the react temperature at 2-8°C,the suspension of condensate was obtained; hydrochloric acid and ice were added to the suspension of condensate, cool to 0-5°C,sodium nitrite was added, reacted at 7-14 °C for 1-2 hours, keeping slight excess of sodium nitrite in the process of reacting , when complete , amino sulfonic acid was added for removing the excess sodium nitrite;
b. the preparation of p-nitroaniline ortho sulfonic acid diazonium salt: p-nitroaniline ortho sulfonic acid and hydrochloric acid were added to water, adding ice to drop to 0-5°C, sodium nitrite solution was added, reacting at 0-5°C for 4-6 hours, when the react was finished, amino sulfonic acid was added for removing the excess nitrous acid;
c. the preparation of H acid solution: H acid was added to water, use 30-40% sodium hydroxide solution neutralize to pH=6-7, make it dissolve;
d. the first step coupling: sodium bicarbonate was added to the diazonium salt of approach a, adjusting pH=1.5-2.5, the H acid of approach c was added, controling the temperature at 10-15°C, reacting for more than 6 hours , red intermediate was obtained;
e. the second step of coupling: diazonium salt react solution of approach b was added to the red intermediate of approach d, using the sodium carbonate to control the pH=5-7, reacted for 2-3 hours , and rised to 30-35°C, 10-20% of the whole volume sodium chloride or potassium chloride was added, stirred and salted out for 2-4 hours ,filtered, the filter cake was the semi-finished product of the black reactive dyes;
f. post treatment: beating and dissolving the semi-finished of approach e, the solution spray drying , obtain reactive black product, orange reactive dyes or red reactive dyes were added, the hight jet black reactive dyes was obtained.

The method of preparing black reactive dyes, including the approaches as follows:
a. the preparation of m-sulfonated ester diazonium salt: m-sulfonated ester and hydrochloric acid were added to water, adding ice to drop to 0-5°C, sodium nitrite solution was added, reacted at 0-5°C for 4-6 hours, when the react was finished, amino sulfonic acid was added for removing the excess nitrous acid;
b. the preparation of dinitroaniline diazonium salt: sodium nitrite was added to 98% sulfuric acid, control the temperature below 30°C, after adding, increased to 65-70°C, keep 2-3 hours; drop the temperature to 30°C below, dinitroaniline was added, control the temperature below 40°C, after adding ,keep 40±3°C for more than 6 hours, drop to 30°C below ,dilute to the mixture of ice and water, the temperature is 2 below , amino sulfonic acid was added for removing the excess sodium nitrite;
c. the first step coupling: directly adding H acid to the diazonium salt of approach a, controlling the temperature at 10-15 , reacting for more than 6 hours . red intermediate was obtained;
d. the second step of coupling: diazonium salt react solution of approach c was added to the red intermediate of approach d, using the sodium carbonate to control the pH=5-7, reacted for 2-3 hours , and rised to 30-35 , 10-20% of the whole volume sodium chloride or potassium chloride was added, stirred and salted out for 2-4 hours ,filtered, the filter cake was the semi-finished product of the black reactive dyes;
e. post treatment: beating and dissolving the semi-finished of approach d, the solution spray drying , obtain reactive black product, orange reactive dyes or red reactive dyes were added, the hight jet black reactive dyes was obtained.

The method of preparing black reactive dyes, including the approaches as follows:
a. the preparation of m-sulfonated ester diazonium salt: m-sulfonated ester and hydrochloric acid were added to water, adding ice to drop to 0-5 , sodium nitrite solution was added, reacted at 0-5 for 4-6 hours, when the react was finished, amino sulfonic acid was added for removing the excess nitrous acid;
b. the preparation of p-nitroaniline diazonium salt: travel milk-12 surfactant, p-nitroaniline, and hydrochloric acid were addedto water, beating for more than 1 hours, ice was added to drop to 0-5 , sodium nitrite solution was added, reacting at 0-5 for 2-3 hours, when the react was finished, amino sulfonic acid was added for removing the excess nitrous acid;
c. the first step coupling: directly adding H acid to the diazonium salt of approach a, controlling the temperature at 10-15 , reacting for more than 6 hours . red intermediate was obtained;
d. the second step of coupling: diazonium salt react solution of approach c was added to the red intermediate of approach d, using the sodium carbonate to control the pH=5-7, reacted for 2-3 hours , and rised to 30-35 , 10-20% of the whole volume sodium chloride or potassium chloride was added, stirred and salted out for 2-4 hours ,filtered, the filter cake was the semi-finished product of the black active dyes;
e. post treatment: beating and dissolving the semi-finished of approach d, the solution spray drying , obtain reactive black product, orange reactive dyes or red reactive dyes were added, the hight jet black reactive dyes was obtained.

The method of preparing black reactive dyes, including the approaches as follows:
a. the preparation of m-sulfonated ester diazonium salt: m-sulfonated ester and hydrochloric acid were added to water, adding ice to drop to 0-5 , sodium nitrite solution was added, reacted at 0-5 for 4-6 hours, when the react was finished, amino sulfonic acid was added for removing the excess nitrous acid;
b. the preparation of p-nitroaniline ortho sulfonic acid diazonium salt: p-nitroaniline ortho sulfonic acid and hydrochloric acid were added to water, adding ice to drop to 0-5 , sodium nitrite solution was added, reacting at 0-5 for 4-6 hours, when the react was finished, amino sulfonic acid was added for removing the excess nitrous acid;
c. the first step coupling: directly adding H acid to the diazonium salt of approach a, controlling the temperature at 10-15 , reacting for more than 6 hours . red intermediate was obtained;
d. the second step of coupling: diazonium salt react solution of approach c was added to the red intermediate of approach d, using the sodium carbonate to control the pH=5-7, reacted for 2-3 hours , and rised to 30-35 , 10-20% of the whole volume sodium chloride or potassium chloride was added, stirred and salted out for 2-4 hours ,filtered, the filter cake was the semi-finished product of the black reactive dyes;
e. post treatment: beating and dissolving the semi-finished of approach d, the solution spray drying , obtain reactive black product, orange reactive dyes or red reactive dyes were added, the hight jet black reactive dyes was obtained.

The present invention is further described by the specific examples.

### Example 1

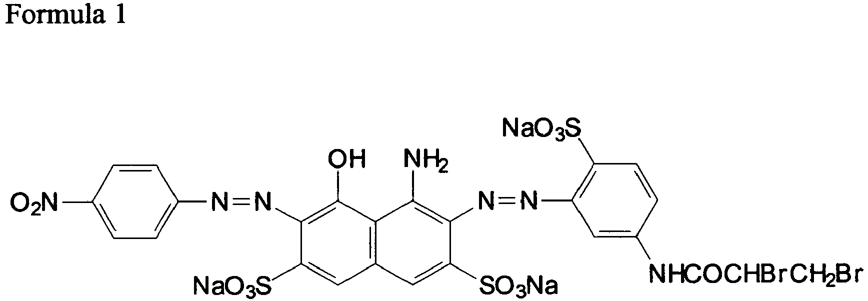

### 1. the preparation of condensate

420kg 2,4-biaminobenzene sulfonic acid sodium was added to water, control the temperature at 2-8 through using ice, 515kg 2,3-bibromopropionyl chloride was added, control the pH=6-8 through using 10-20% sodium carbonate solution, react for 3-6 hours, control the react temperature at 2-8 ,obtain the suspension of condensate, reserve.

### 2. the preparation of condensate diazonium salt

560kg 30%hydrochloric acid was added to the suspension of condensate, ice was added to cool to 0-5 ,and 138kg 30-40% sodium nitrite solution was added, react at 7-14 for 1-2 hours. when complete , H₂NSO₃H (amino sulfonic acid) was added for removing the excess sodium nitrite.

### 3. the preparation of p-nitroaniline diazonium salt

12kg travel milk-12 surfactant, 238.6kg p-nitroaniline, and 543kg 30%hydrochloric acid were added to 7500L water, beating for more than 1 hours, ice was added to drop to 0-5 , 30-40% solution containing 138kg sodium nitrite was added, reacting at 0-5 for 2-3 hours, when the react was finished, amino sulfonic acid was added for removing the excess nitrous acid.

### 4. the preparation of H acid solution:

661kg H acid was added to 7500L water, using 30-40% sodium hydroxide solution neutralize to pH=6-7, make it dissolve;

### 5. the first step coupling:

sodium bicarbonate was added to the diazonium salt of approach 2, adjust pH=1.5-2.5, add the H acid of approach , control the temperature at 10-15 , react for more than 6 hours , the red intermediate was obtained;

### 6. the second step of coupling:

diazonium salt react solution of approach 3 was added to the red intermediate of approach 5, using the sodium carbonate to control the pH=5-7, reacted for 2-4 hours , rised to 30-35 , 10-20% of the whole volume sodium chloride was added, stirred and salted out for 2-4 hours ,filtered, the filter cake was the semi-finished product of the black reactive dyes of formula 1.

### 7.post treatment

beating and dissolving the semi-finished of approach 6, the solution spray drying , obtain reactive black product. orange reactive dyes or red reactive dyes were added, the hight jet black black reactive dyes was obtained.

### Example 2

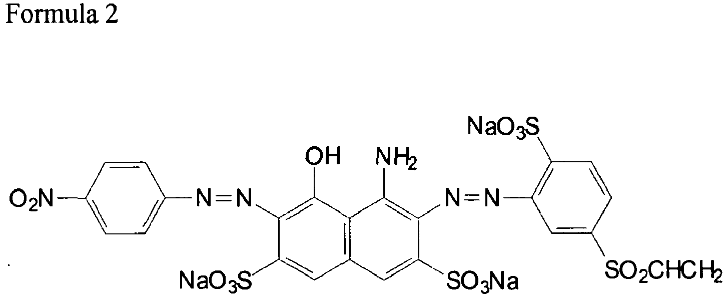

### 1. the preparation of p-nitroaniline diazonium salt

12kg travel milk-12 surfactant,238.6kg p-nitroaniline, and 543kg 30%hydrochloric acid were added to 7500L water, beating for more than 1 hours, ice was added to drop to 0-5 , 30-40% solution containing 138kg sodium nitrite was added, reacting at 0-5 for 2-3 hours, when the react was finished, amino sulfonic acid was added for removing the excess nitrous acid.

### 2. the preparation of m-sulfonated ester diazonium salt

722kg m-sulfonated ester and 317kf 30% hydrochloric acid were added to 7500L water, adding ice to drop to 0-5 , 30-40% solution containing 138kg sodium nitrite was added, reacted at 0-5 for 4-6 hours, when the react was finished, amino sulfonic acid was added for removing the excess nitrous acid.

### 3. the first step coupling:

directly adding H acid to the diazonium salt of approach 2, controlling the temperature at 10-15 , reacting for more than 6 hours . red intermediate was obtained;

### 4. the second step of coupling:

diazonium salt react solution of approach 1 was added to the red intermediate of approach 3, using the sodium carbonate to control the pH=5-7, reacted for 2-4 hours ,cooled to 0-5 , using 30-40% sodium hydroxide solution to adjust to pH=11-12, keeped for 1-2 hours . Adjusted to pH=6-7 with 30% hydrochloric acid and rised to 30-35 , 10-20% of the whole volume sodium chloride was added, stirred and salted out for 2-4 hours ,filtered, the filter cake was the semi-finished product of the black active dyes of formula 2.

### 5. post treatment

beating and dissolving the semi-finished of approach 4, the solution spray drying , obtaining reactive black product. orange reactive dyes or red reactive dyes were added, the hight jet black reactive dyes was obtained.

### Example 3

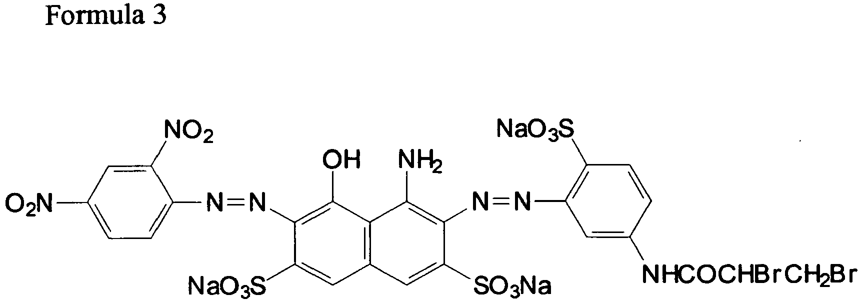

### 1. the preparation of condensate

420kg 2,4-biaminobenzene sulfonic acid sodium was added to water, control the temperature at 2-8 through using ice, 515kg 2,3-bibromopropionyl chloride was added, control the pH=6-8 through using 10-20% sodium carbonate solution, react for 3-6 hours, control the react temperature at 2-8 ,obtain the suspension of condensate, reserve.

### 2. the preparation of condensate diazonium salt

560kg 30%hydrochloric acid was added to the suspension of condensate, ice was added to cool to 0-5 ,and 138kg 30-40% sodium nitrite solution was added, react at 7-14 for 1-2 hours. when complete , H₂NSO₃H (amino sulfonic acid) was added for removing the excess sodium nitrite.

### 3.the preparation of 2.4-dinitrodiazonium salt

165kg sodium nitrite was added to 1800kg 98% sulfuric acid, control the temperature below 30 , after adding, increased to 65-70 , keep 2-3 hours. drop the temperature to 30 below, 302kg 2.4-dinitroaniline was added, control the temperature below 40 , after adding ,keep 40±3 for more than 6 hours. drop to 30 below ,dilute to the mixture of ice and water, the temperature is 2 below , H₂NSO₃H(amino sulfonic acid) was added for removing the excess sodium nitrite.

### 4. the preparation of H acid solution:

661kg H acid was added to 7500L water, using 30-40% sodium hydroxide solution neutralize to pH=6-7, make it dissolve;

### 5. the first step coupling:

sodium bicarbonate was added to the diazonium salt of approach 2, adjust pH=1.5-2.5, add the H acid of approach , control the temperature at 10-15 , react for more than 6 hours , the red intermediate was obtained;

### 6. the second step of coupling:

diazonium salt react solution of approach 3 was added to the red intermediate of approach 5, using the sodium carbonate to control the pH=5-7, reacted for 2-4 hours , rised to 30-35 , 10-20% of the whole volume sodium chloride was added, stirred and salted out for 2-4 hours ,filtered, the filter cake was the semi-finished product of the black reactive dyes of formula 1.

### 7.post treatment

beating and dissolving the semi-finished of approach 6, the solution spray drying , obtain reactive black product. orange reactive dyes or red active dyes were added, the hight jet black black reactive dyes was obtained.

### Example 4

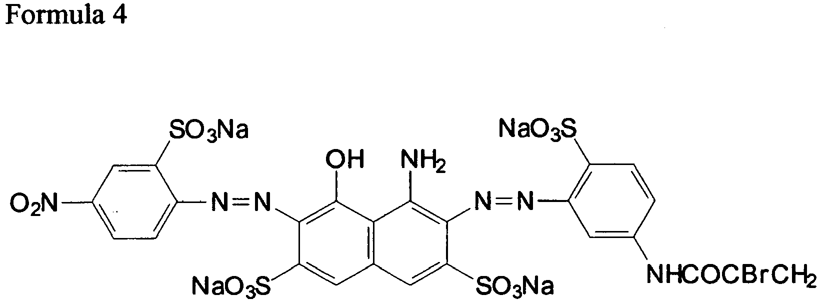

### 1. the preparation of condensate

420kg 2,4-biaminobenzene sulfonic acid sodium was added to water, control the temperature at 2-8 through using ice, 515kg 2,3-bibromopropionyl chloride was added, control the pH=6-8 through using 10-20% sodium carbonate solution, react for 3-6 hours, control the react temperature at 2-8 ,obtain the suspension of condensate, reserve.

### 2. the preparation of condensate diazonium salt

560kg 30%hydrochloric acid was added to the suspension of condensate, ice was added to cool to 0-5 ,and 138kg 30-40% sodium nitrite solution was added, react at 7-14 for 1-2 hours. when complete , H₂NSO₃H (amino sulfonic acid) was added for removing the excess sodium nitrite.

### 3. the preparation of p-nitroaniline diazonium salt

408kg p-nitroaniline and 317kg 30% hydrochloric acid were added to 7500L water, ice was added to cool to 0-5 ,and 138kg 30-40% sodium nitrite solution was added, react at 0-5 for 2-3 hours. when complete , amino sulfonic acid was added for removing the excess sodium nitrite.

### 4. the preparation of H acid solution:

661kg H acid was added to 7500L water, using 30-40% sodium hydroxide solution neutralize to pH=6-7, make it dissolve;

### 5. the first step coupling:

sodium bicarbonate was added to the diazonium salt of approach 2, adjust pH=1.5-2.5, add the H acid of approach , control the temperature at 10-15 , react for more than 6 hours , the red intermediate was obtained;

### 6. the second step of coupling:

diazonium salt react solution of approach 3 was added to the red intermediate of approach 5, using the sodium carbonate to control the pH=5-7, reacted for 2-4 hours ,cooled to 0-5 , using 30-40% sodium hydroxide solution to adjust to pH=11-12, keeped for 1-2 hours . Adjusted to pH=6-7 with 30% hydrochloric acid and rised to 30-35 , 10-20% of the whole volume sodium chloride was added, stirred and salted out for 2-4 hours ,filtered, the filter cake was the semi-finished product of the black reactive dyes of formula 2.

### 7. post treatment

beating and dissolving the semi-finished of approach 6, the solution spray drying , obtaining reactive black product. orange reactive dyes or red reactive dyes were added, the hight jet black reactive dyes was obtained.

When dyeing the fiber, the product of the present invention is aquamarine blue, the maximum absorption wavelength is 606nm, at 750nm it has obviously absorption. When adding appropriate of red compositions or orange compositons, hight jet black active black product can be obtained, when using it can stain without adding any heave metals as the addtivites, it can replace the acid mordant black PV used for staining the cashmere and other worsted fabric, it also can be used for staining the fiber, such as cotton, silk, polyamide and so on. The method avoid the product of printing and dyeing wastewater containing heavy metal Cr6⁺, and promote technological progress of printing and dyeing fields.

## Claims

1. A kind of black reactive dyes, represented by the formula 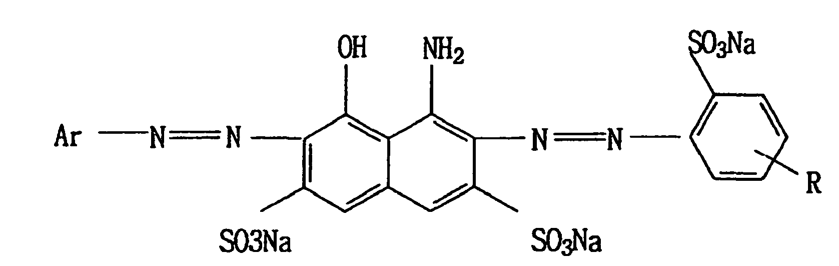 Where
R=-NHCOCHBrCH₂Br -NHCOCBrCH₂ or -SO₂CH=CH₂

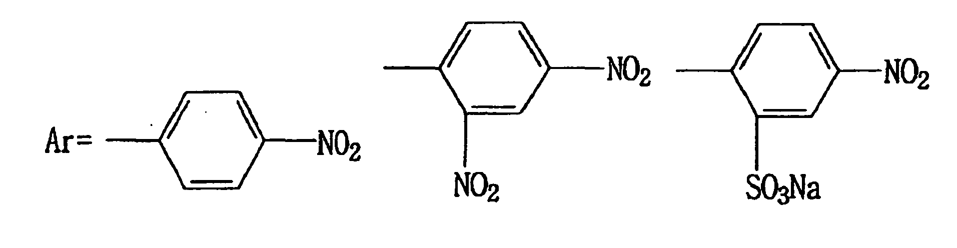

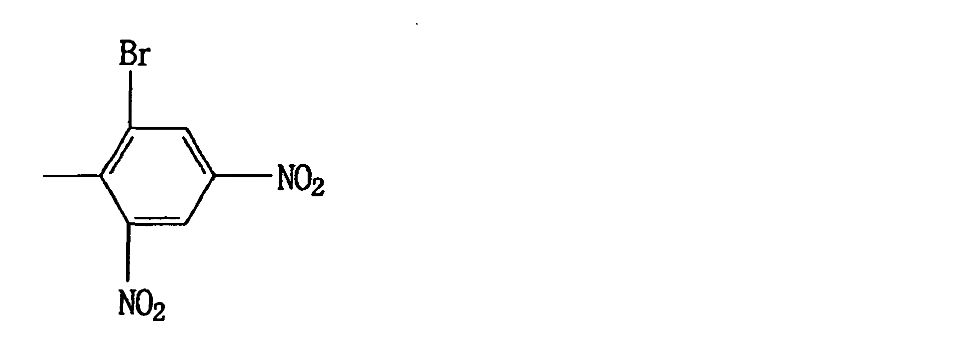

2. The method of preparing black reactive dyes according to claim 1, including the approaches as follows:
a. the preparation of condensate diazonium salt: 2,4-biaminobenzene sulfonic acid sodium was added to water, control the temperature at 2-8°C through using ice, 2,3-bibromopropionyl chloride was added, control the pH=6-8 through using 10-20% sodium carbonate solution, react for 3-6 hours, control the react temperature at 2-8°C,the suspension of condensate was obtained; hydrochloric acid and ice were added to the suspension of condensate, cool to 0-5°C,sodium nitrite was added, reacted at 7-14 °C for 1-2 hours, keeping slight excess of sodium nitrite in the process of reacting , when complete , amino sulfonic acid was added for removing the excess sodium nitrite;
b. the preparation of dinitroaniline diazonium salt: sodium nitrite was added to 98% sulfuric acid, control the temperature below 30°C, after adding, increased to 65-70°C, keep 2-3 hours; drop the temperature to 30°C below, dinitroaniline was added, control the temperature below 40°C, after adding ,keep 40+3°C for more than 6 hours, drop to 30°C below ,dilute to the mixture of ice and water, the temperature is 2°C below , amino sulfonic acid was added for removing the excess sodium nitrite;
c. the preparation of H acid solution: quantitative H acid was added to water, use 30-40% sodium hydroxide solution neutralize to pH=6-7, make it dissolve;
d. the first step coupling: sodium bicarbonate was added to the diazonium salt of approach a, adjust pH=1.5-2.5, add the H acid of approach , control the temperature at 10-15°C, react for more than 6 hours , obtain red intermediate;
e. the second step of coupling: diazonium salt react solution of approach b was added to the red intermediate of approach d, using the sodium carbonate to control the pH=5-7, reacted for 2-3 hours, raised to 30-35°C, 10-20% of the whole volume sodium chloride or potassium chloride was added, stir and salt out for 2-4 hours, filter, the filter cake is the semi-finished product of the black reactive dyes;
f. post treatment: beating and dissolving the semi-finished of approach e, the solution spray drying , obtain reactive black product, orange reactive dyes or red reactive dyes were added, the high jet black reactive dyes was obtained.

3. The method of preparing black reactive dyes according to claim 1, including the approaches as follows:
a. the preparation of condensate diazonium salt: 2,4-biaminobenzene sulfonic acid sodium was added to water, control the temperature at 2-8°C through using ice, 2,3-bibromopropionyl chloride was added, control the pH=6-8 through using 10-20% sodium carbonate solution, react for 3-6 hours, control the react temperature at 2-8°C,obtain the suspension of condensate; hydrochloric acid and ice were added to the suspension of condensate, cool to 0-5°C,add sodium nitrite, react at 7-14 °C for 1-2 hours, keep slight excess of sodium nitrite in the process of reacting , when complete , amino sulfonic acid was added for removing the excess sodium nitrite;
b. the preparation of p-nitroaniline diazonium salt: travel milk-12 surfactant, p-nitroaniline, and hydrochloric acid were added to water, beating for more than 1 hours, ice was added to drop to 0-5°C, sodium nitrite solution was added, reacting at 0-5°C for 2-3 hours, when the react was finished, amino sulfonic acid was added for removing the excess nitrous acid;
c. the preparation of H acid solution: H acid was added to water, use 30-40% sodium hydroxide solution neutralize to pH=6-7, make it dissolve;
d. the first step coupling: sodium bicarbonate was added to the diazonium salt of approach a, adjusting pH=1.5-2.5, the H acid of approach c was added, controlling the temperature at 10-15°C, reacting for more than 6 hours , red intermediate was obtained;
e. the second step of coupling: diazonium salt react solution of approach b was added to the red intermediate of approach d, using the sodium carbonate to control the pH=5-7, reacted for 2-3 hours , and raised to 30-35°C, 10-20% of the whole volume sodium chloride or potassium chloride was added, stirred and salted out for 2-4 hours ,filtered, the filter cake was the semi-finished product of the black reactive dyes;
f. post treatment: beating and dissolving the semi-finished of approach e, the solution spray drying , obtain reactive black product, orange reactive dyes or red reactive dyes were added, the high jet black reactive dyes was obtained.

4. The method of preparing black reactive dyes according to claim 1, including the approaches as follows:
a. the preparation of condensate diazonium salt: 2,4-biaminobenzene sulfonic acid sodium was added to water, control the temperature at 2-8°C through using ice, 2,3-bibromopropionyl chloride was added, control the pH=6-8 through using 10-20% sodium carbonate solution, react for 3-6 hours, control the react temperature at 2-8°C,the suspension of condensate was obtained; hydrochloric acid and ice were added to the suspension of condensate, cool to 0-5°C,sodium nitrite was added, reacted at 7-14 °C for 1-2 hours, keeping slight excess of sodium nitrite in the process of reacting , when complete , amino sulfonic acid was added for removing the excess sodium nitrite;
b. the preparation of p-nitroaniline ortho sulfonic acid diazonium salt: p-nitroaniline ortho sulfonic acid and hydrochloric acid were added to water, adding ice to drop to 0-5°C, sodium nitrite solution was added, reacting at 0-5°C for 4-6 hours, when the react was finished, amino sulfonic acid was added for removing the excess nitrous acid;
c. the preparation of H acid solution: H acid was added to water, use 30-40% sodium hydroxide solution neutralize to pH=6-7, make it dissolve;
d. the first step coupling: sodium bicarbonate was added to the diazonium salt of approach a, adjusting pH=1.5-2.5, the H acid of approach c was added, controlling the temperature at 10-15°C, reacting for more than 6 hours , red intermediate was obtained;
e. the second step of coupling: diazonium salt react solution of approach b was added to the red intermediate of approach d, using the sodium carbonate to control the pH=5-7, reacted for 2-3 hours , and raised to 30-35°C, 10-20% of the whole volume sodium chloride or potassium chloride was added, stirred and salted out for 2-4 hours ,filtered, the filter cake was the semi-finished product of the black reactive dyes;
f. post treatment: beating and dissolving the semi-finished of approach e, the solution spray drying , obtain reactive black product, orange reactive dyes or red reactive dyes were added, the high jet black reactive dyes was obtained.

5. The method of preparing black reactive dyes according to claim 1, including the approaches as follows:
a. the preparation of m-sulfonated ester diazonium salt: m-sulfonated ester and hydrochloric acid were added to water, adding ice to drop to 0-5°C, sodium nitrite solution was added, reacted at 0-5°C for 4-6 hours, when the react was finished, amino sulfonic acid was added for removing the excess nitrous acid;
b. the preparation of dinitroaniline diazonium salt: sodium nitrite was added to 98% sulfuric acid, control the temperature below 30°C, after adding, increased to 65-70°C, keep 2-3 hours; drop the temperature to 30°C below, dinitroaniline was added, control the temperature below 40°C, after adding ,keep 40+3°C for more than 6 hours, drop to 30°C below ,dilute to the mixture of ice and water, the temperature is 2°C below , amino sulfonic acid was added for removing the excess sodium nitrite;
c. the first step coupling: directly adding H acid to the diazonium salt of approach a, controlling the temperature at 10-15°C, reacting for more than 6 hours . red intermediate was obtained;
d. the second step of coupling: diazonium salt react solution of approach c was added to the red intermediate of approach d, using the sodium carbonate to control the pH=5-7, reacted for 2-3 hours, and raised to 30-35°C, 10-20% of the whole volume sodium chloride or potassium chloride was added, stirred and salted out for 2-4 hours ,filtered, the filter cake was the semi-finished product of the black reactive dyes;
e. post treatment: beating and dissolving the semi-finished of approach d, the solution spray drying , obtain reactive black product, orange reactive dyes or red reactive dyes was added, the high jet black reactive dyes was obtained.

6. The method of preparing black reactive dyes according to claim 1, including the approaches as follows:
a. the preparation of m-sulfonated ester diazonium salt: m-sulfonated ester and hydrochloric acid were added to water, adding ice to drop to 0-5°C, sodium nitrite solution was added, reacted at 0-5°C for 4-6 hours, when the react was finished, amino sulfonic acid was added for removing the excess nitrous acid;
b. the preparation of p-nitroaniline diazonium salt: travel milk-12 surfactant, p-nitroaniline, and hydrochloric acid were added to water, beating for more than 1 hours, ice was added to drop to 0-5°C, sodium nitrite solution was added, reacting at 0-5°C for 2-3 hours, when the react was finished, amino sulfonic acid was added for removing the excess nitrous acid;
c. the first step coupling: directly adding H acid to the diazonium salt of approach a, controlling the temperature at 10-15°C, reacting for more than 6 hours . red intermediate was obtained;
d. the second step of coupling: diazonium salt react solution of approach c was added to the red intermediate of approach d, using the sodium carbonate to control the pH=5-7, reacted for 2-3 hours, and raised to 30-35°C, 10-20% of the whole volume sodium chloride or potassium chloride was added, stirred and salted out for 2-4 hours ,filtered, the filter cake was the semi-finished product of the black reactive dyes;
e. post treatment: beating and dissolving the semi-finished of approach d, the solution spray drying , obtain reactive black product, orange reactive dyes or red reactive dyes were added, the high jet black reactive dyes was obtained.

7. The method of preparing black reactive dyes according to claim 1, including the approaches as follows:
a. the preparation of m-sulfonated ester diazonium salt: m-sulfonated ester and hydrochloric acid were added to water, adding ice to drop to 0-5°C, sodium nitrite solution was added, reacted at 0-5°C for 4-6 hours, when the react was finished, amino sulfonic acid was added for removing the excess nitrous acid;
b. the preparation of p-nitroaniline ortho sulfonic acid diazonium salt: p-nitroaniline ortho sulfonic acid and hydrochloric acid were added to water, adding ice to drop to 0-5°C, sodium nitrite solution was added, reacting at 0-5°C for 4-6 hours, when the react was finished, amino sulfonic acid was added for removing the excess nitrous acid;
c. the first step coupling: directly adding H acid to the diazonium salt of approach a, controlling the temperature at 10-15°C, reacting for more than 6 hours red intermediate was obtained;
d. the second step of coupling: diazonium salt react solution of approach c was added to the red intermediate of approach d, using the sodium carbonate to control the pH=5-7, reacted for 2-3 hours , and raised to 30-35°C, 10-20% of the whole volume sodium chloride or potassium chloride was added, stirred and salted out for 2-4 hours ,filtered, the filter cake was the semi-finished product of the black reactive dyes;
e. post treatment: beating and dissolving the semi-finished of approach d, the solution spray drying , obtain reactive black product, orange reactive dyes or red reactive dyes were added, the high jet black reactive dyes was obtained.

## Patentansprüche

1. Art schwarzer Reaktivfarbstoffe, dargestellt durch die Formel 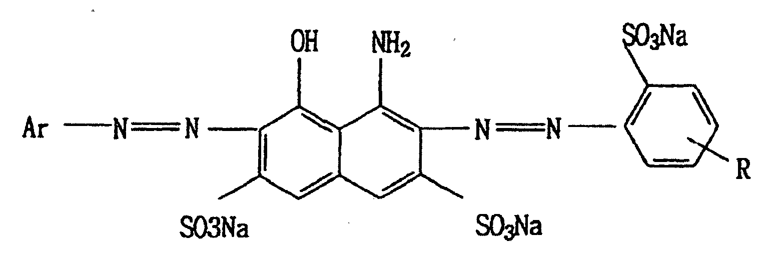 worin R = -NHCOCHBrCH₂Br, -NHCOCBrCH₂ oder -SO₂CH=CH₂

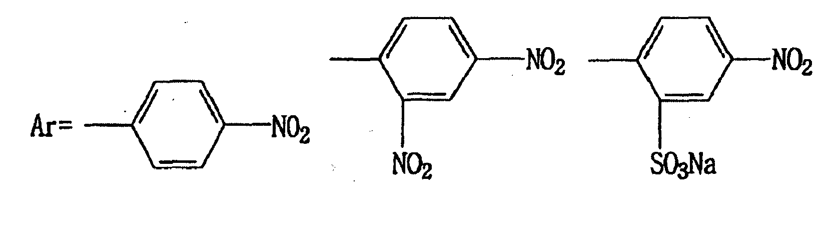

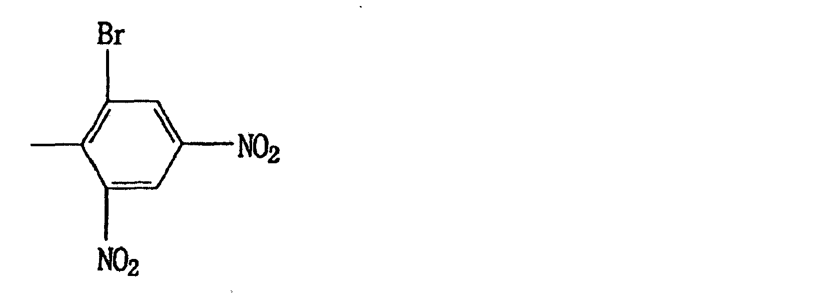

2. Verfahren zur Herstellung schwarzer Reaktivfarbstoffe gemäß Anspruch 1, umfassend die Ansätze wie folgt:
a. Herstellung von Diazoniumsalz-Kondensat: 2,4-Biaminobenzolsulfonsäure-natrium wurde zu Wasser gegeben, die Temperatur wurde durch Verwendung von Eis auf 2 bis 8 °C reguliert, 2,3-Bibrompropionylchlorid wurde zugegeben, der pH = 6-8 wurde durch Verwendung von 10-20 % Natriumcarbonatlösung reguliert, es wurde für 3-6 Stunden umgesetzt, die Reaktionstemperatur wurde auf 2-8 °C reguliert, es wurde die Suspension von Kondensat erhalten; Salzsäure und Eis wurden zu der Suspension von Kondensat gegeben, es wurde auf 0-5 °C gekühlt, Natriumnitrit wurde zugesetzt, es wurde bei 7-14 °C für 1-2 Stunden umgesetzt, wobei ein leichter Überschuss an Natriumnitrit im Reaktionsverfahren aufrechterhalten wurde, als dieses beendet war, wurde Aminosulfonsäure zur Entfernung des überschüssigen Natriumnitrits zugesetzt;
b. Herstellung von Dinitroanilin-Diazoniumsalz: Natriumnitrit wurde zu 98 % Schwefelsäure gegeben, die Temperatur wurde unter 30 °C reguliert, nach dem Zusetzen auf 65-70 °C erhöht, für 2-3 Stunden gehalten; die Temperatur wurde auf unter 30 °C gesenkt, Dinitroanilin wurde zugesetzt, die Temperatur wurde auf unter 40 °C reguliert, nach Zusetzen wurde sie für mehr als 6 Stunden auf 40+3 °C gehalten, auf unter 30 °C gesenkt, es wurde mit einem Gemisch aus Eis und Wasser verdünnt, als die Temperatur unter 2 °C war, wurde Aminosulfonsäure zur Entfernung des überschüssigen Natriumnitrits zugesetzt;
c. Herstellung von H-Säure-Lösung: quantitative H-Säure wurde zu Wasser gegeben, es wurde 30-40 % Natriumhydroxidlösung verwendet, um auf pH = 6-7 zu neutralisieren, es in Lösung gebracht;
d. erster Kupplungsschritt: Natriumbicarbonat wurde zu dem Diazoniumsalz von Ansatz a gegeben, pH = 1,5-2,5 wurde eingestellt, die H-Säure vom Ansatz zugesetzt, die Temperatur auf 10-15 °C reguliert, es wurde für mehr als 6 Stunden umgesetzt, wodurch ein rotes Intermediat erhalten wurde;
e. zweiter Kupplungsschritt: Diazoniumsalz-Reaktionslösung von Ansatz b wurde zu dem roten Intermediat von Ansatz d gegeben, es wurde Natriumcarbonat verwendet, um den pH = 5-7 einzustellen, es wurde für 2-3 Stunden umgesetzt, die Temperatur wurde auf 30-35 °C erhöht, 10-20 % des gesamten Volumens an Natriumchlorid oder Kaliumchlorid wurde zugegeben, es wurde für 2-4 Stunden gerührt und ausgesalzt, filtriert, der Filterkuchen war das halbfertige Produkt der schwarzen Reaktivfarbstoffe;
f. Nachbehandlung: Mahlen und Lösen des halbfertigen Produktes von Ansatz e, Sprühtrocknen der Lösung unter Erhalt von schwarzem Reaktivfarbstoffprodukt, orangefarbene Reaktivfarbstoffe oder rote Reaktivfarbstoffe wurden zugesetzt, wobei die schwarzen "high jet"-Reaktivfarbstoffe erhalten wurden.

3. Verfahren zur Herstellung schwarzer Reaktivfarbstoffe gemäß Anspruch 1, umfassend die Ansätze wie folgt:
a. Herstellung von Diazoniumsalz-Kondensat: 2,4-Biaminobenzolsulfonsäure-natrium wurde zu Wasser gegeben, die Temperatur wurde durch Verwendung von Eis auf 2-8 °C reguliert, 2,3-Bibrompropionylchlorid wurde zugesetzt, der pH wurde durch Verwendung von 10-20 % Natriumcarbonatlösung auf pH = 6-8 reguliert, es wurde für 3-6 Stunden umgesetzt, die Reaktionstemperatur wurde auf 2-8 °C reguliert, die Suspension von Kondensat wurde erhalten; Salzsäure und Eis wurden zu der Suspension von Kondensat gegeben, es wurde auf 0-5 °C gekühlt, es wurde Natriumnitrit zugegeben, es wurde für 1-2 Stunden bei 7-14 °C umgesetzt, wobei ein leichter Überschuss an Natriumnitrit im Reaktionsverfahren aufrechterhalten wurde, wobei, als dieses beendet war, Aminosulfonsäure zur Entfernung des überschüssigen Natriumnitrits zugesetzt wurde;
b. Herstellung von p-Nitroanilin-Diazoniumsalz: "travel milk-12 surfactant", p-Nitroanilin und Salzsäure wurden zu Wasser gegeben, es wurde für mehr als 1 Stunde gemahlen, Eis wurde zugesetzt, um auf 0-5 °C zu kühlen, Natriumnitritlösung wurde zugesetzt, es wurde bei 0-5 °C für 2-3 Stunden umgesetzt, als die Reaktion beendet war, wurde Aminosulfonsäure zur Entfernung der überschüssigen salpetrigen Säure zugesetzt;
c. Herstellung von H-Säure-Lösung: H-Säure wurde zu Wasser gegeben, es wurde 30-40 % Natriumhydroxidlösung verwendet, um auf pH 6-7 zu neutralisieren, es wurde in Lösung gebracht;
d. erster Kupplungsschritt: Natriumbicarbonat wurde zu dem Diazoniumsalz von Ansatz a gegeben, der pH wurde auf 1,5-2,5 eingestellt, die H-Säure von Ansatz c wurde zugesetzt, die Temperatur wurde auf 10-15 °C reguliert, es wurde für mehr als 6 Stunden reagieren gelassen, es wurde rotes Intermediat erhalten;
e. zweiter Kupplungsschritt: Diazoniumsalz-Reaktionslösung von Ansatz b wurde zu dem roten Intermediat von Ansatz d gegeben, es wurde Natriumcarbonat verwendet, um den pH = 5-7 zu regulieren, es wurde für 2-3 Stunden umgesetzt und die Temperatur wurde auf 30-35 °C erhöht, 10-20 % des gesamten Volumens an Natriumchlorid oder Kaliumchlorid wurden zugesetzt, es wurde für 2-4 Stunden gerührt und ausgesalzt, filtriert, der Filterkuchen war das halbfertige Produkt der schwarzen Reaktivfarbstoffe;
f. Nachbehandlung: Mahlen und Lösen des halbfertigen Produktes von Ansatz e, die Lösung wurde sprühgetrocknet, wodurch schwarzes Reaktivfarbstoffprodukt erhalten wurde, orangefarbene Reaktivfarbstoffe oder rote Reaktivfarbstoffe wurden zugesetzt, die schwarzen "high-jet"-Reaktivfarbstoffe wurden erhalten.

4. Verfahren zur Herstellung schwarzer Reaktivfarbstoffe gemäß Anspruch 1, umfassend die Ansätze wie folgt:
a. Herstellung von Diazoniumsalz-Kondensat: 2,4-Biaminobenzolsulfonsäure-natrium wurde zu Wasser gegeben, die Temperatur wurde unter Verwendung von Eis auf 2-8 °C reguliert, 2,3-Bibrompropionylchlorid wurde zugesetzt, der pH = 6-8 wurde durch Verwendung von 10-20 % Natriumcarbonatlösung reguliert, es wurde für 3-6 Stunden umgesetzt, die Reaktionstemperatur wurde auf 2-8 °C reguliert, die Suspension von Kondensat wurde erhalten; Salzsäure und Eis wurden zu der Suspension von Kondensat gegeben, es wurde auf 0-5 °C gekühlt, Natriumnitrit wurde zugegeben, es wurde bei 7-14 °C für 1-2 Stunden umgesetzt, wobei ein leichter Überschuss an Natriumnitrit im Reaktionsverfahren aufrechterhalten wurde, wobei, als dieses beendet war, Aminosulfonsäure zur Entfernung des überschüssigen Natriumnitrits zugesetzt wurde;
b. Herstellung von p-Nitroanilin-ortho-sulfonsäure-Diazoniumsalz: p-Nitroanilin-ortho-sulfonsäure und Salzsäure wurden zu Wasser gegeben, es wurde Eis zugesetzt, um auf 0-5 °C zu kühlen, Natriumnitritlösungn wurde zugesetzt, es wurde bei 0-5 °C für 4-6 Stunden umgesetzt, wobei, als die Reaktion beendet war, Aminosulfonsäure zur Entfernung der überschüssigen salpetrigen Säure zugesetzt wurde;
c. Herstellung von H-Säure-Lösung: H-Säure wurde zu Wasser gegeben, es wurde 30-40 % Natriumhydroxidlösung verwendet, um auf pH = 6-7 zu neutralisieren, es wurde in Lösung gebracht;
d. erster Kupplungsschritt: Natriumbicarbonat wurde zu dem Diazoniumsalz von Ansatz a gegeben, es wurde ein pH = 1,5-2,5 eingestellt, die H-Säure von Ansatz c wurde zugesetzt, die Temperatur wurde auf 10-15 °C reguliert, es wurde für mehr als 6 Stunden umgesetzt, rotes Intermediat wurde erhalten;
e. zweiter Kupplungsschritt: Diazoniumsalz-Reaktionslösung von Ansatz b wurde zu dem roten Intermediat von Ansatz d gegeben, Natriumcarbonat wurde verwendet, um den pH = 5-7 zu kontrollieren, es wurde für 2-3 Stunden umgesetzt und die Temperatur wurde auf 30-35 °C erhöht, 10-20 % des gesamten Volumens an Natriumchlorid oder Kaliumchlorid wurde zugesetzt, es wurde für 2-4 Stunden gerührt und ausgesalzt, filtriert, der Filterkuchen war das halbfertige Produkt der schwarzen Reaktivfarbstoffe;
f. Nachbehandlung: Mahlen und Lösen des halbfertigen Produktes von Ansatz e, Sprühtrocknen der Lösung, es wurde schwarzes Reaktivfarbstoffprodukt erhalten, orangefarbene Reaktivfarbstoffe oder rote Reaktivfarbstoffe wurden zugesetzt, es wurden schwarze "high jet" -Reaktivfarbstoffe erhalten.

5. Verfahren zur Herstellung schwarzer Reaktivfarbstoffe gemäß Anspruch 1, umfassend die Ansätze wie folgt:
a. Herstellung von m-sulfoniertem Ester-Diazoniumsalz: m-sulfonierter Ester und Salzsäure wurden zu Wasser gegeben, es wurde Eis zugesetzt, um die Temperatur auf 0-5 zu senken, es wurde Natriumnitritlösung zugesetzt, es wurde bei 0-5 °C für 4-6 Stunden umgesetzt, wobei, als die Reaktion beendet war, Aminosulfonsäure zur Entfernung der überschüssigen salpetrigen Säure zugesetzt wurde;
b. Herstellung von Dinitroanilin-Diazoniumsalz: Natriumnitrit wurde zu 98 % Schwefelsäure gegeben, die Temperatur wurde auf unter 30 °C reguliert, nach Zugabe auf 65-70 °C erhöht, 2-3 Stunden gehalten; die Temperatur wurde auf unter 30 °C gesenkt, Dütroanilin wurde zugesetzt, die Temperatur wurde auf unter 40 °C reguliert, nach Zugeben wurde sie für mehr als 6 Stunden bei 40+3 °C gehalten, sie wurde auf 30 °C gesenkt, das Gemisch wurde mit Eis und Wasser verdünnt, wenn die Temperatur unter 2 °C war, wurde Aminosulfonsäure zur Entfernung von überschüssigem Natriumnnitrit zugesetzt;
c. erster Kupplungsschritt: direktes Zugeben von H-Säure zu dem Diazoniumsalz von Ansatz a, Regulieren der Temperatur auf 10-15 °C, Umsetzen für mehr als 6 Stunden, rotes Intermediat wurde erhalten;
d. zweiter Kupplungsschritt: Diazonium-Reaktionslösung von Ansatz c wurde zu dem rotem Intermediat von Ansatz d gegeben, wobei Natiumcarbonat verwendet wurde, um den pH = 5-7 zu regulieren, es wurde für 2-3 Stunden umgesetzt und die Temperatur wurde auf 30-35 °C erhöht, 10-20 % des gesamten Volumens an Natriumchlorid oder Kaliumchlorid wurden zugegeben, es wurde für 2-4 Stunden gerührt und ausgesalzt, filtriert, der Filterkuchen war das halbfertige Produkt der schwarzen Reaktivfarbstoffe;
e. Nachbehandlung: Mahlen und Lösen des halbfertigen Produktes von Ansatz d, Sprühtrocknen der Lösung, wobei schwarzes Reaktivfarbstoffprodukt erhalten wurde, orangefarbene Reaktivfarbstoffe oder rote Reaktivfarbstoffe wurden zugesetzt, es wurden schwarze "high jet"-Reaktivfarbstoffe erhalten.

6. Verfahren zur Herstellung schwarzer Reaktivfarbstoffe gemäß Anspruch 1, umfassend die Ansätze wie folgt:
a. Herstellung von m-sulfoniertem Ester-Diazoniumsalz: m-sulfonierter Ester und Salsäure wurden zu Wasser gegeben, es wurde Eis zugesetzt, um die Temperatur auf 0-5 °C zu senken, Natriumnitritlösung wurde zugesetzt, es wurde für 4-6 Stunden bei 0-5 °C umgesetzt, als die Reaktion beendet war, wurde Aminosulfonsäure zur Entfernung der überschüssigen salpetrigen Säure zugesetzt;
b. Herstellung von p-Nitroanilin-Diazoniumsalz: "travel milk-12 surfactant", p-Nitroanilin und Salzsäure wurden zu Wasser gegeben, es wurde für mehr als 1 Stunde gemahlen, Eis wurde zugesetzt, um die Temperatur auf 0-5 °C zu senken, Natriumnitritlösung wurde zugesetzt, es wurde für 2-3 Stunden bei 0-5 °C umgesetzt, als die Reaktion beendet war, wurde Aminosulfonsäure zur Entfernung der überschüssigen salpetrigen Säure zugesetzt;
c. erster Kupplungsschritt: direktes Zugeben von H-Säure zu dem Diazoniumsalz von Ansatz a, Regulieren der Temperatur auf 10-15 °C, Umsetzen für mehr als 6 Stunden, es wurde rotes Intermediat erhalten;
d. zweiter Kupplungsschritt: Diazoniumsalz-Reaktionslösung von Ansatz c wurde zu dem roten Intermediat von Ansatz d gegeben, wobei Natriumcarbonat verwendet wurde, um den pH = 5-7 zu regulieren, es wurde für 2-3 Stunden umgesetzt und die Temperatur wurde auf 30-35 °C erhöht, 10-20 % des gesamten Volumens an Natriumchlorid oder Kaliumchlorid wurden zugesetzt, es wurde für 2-4 Stunden gerührt und ausgesalzt, filtriert, der Filterkuchen war das halbfertige Produkt der schwarzen Reaktivfarbstoffe;
e. Nachbehandlung: Mahlen und Lösen des halbfertigen Produktes von Ansatz d, Sprühtrocknen der Lösung, wobei das schwarze Reaktivfarbstoffprodukt erhalten wird, orangefarbener Reaktivfarbstoffe oder rote Reaktivfarbstoffe wurden zugesetzt, es wurden schwarze "high jet"-Reaktivfarbstoffe erhalten.

7. Verfahren zur Herstellung schwarzer Reaktivfarbstoffe gemäß Anspruch 1, umfassend die Ansätze wie folgt:
a. Herstellung von m-sulfoniertem Ester-Diazoniumsalz: m-sulfonierter Ester und Salzsäure wurden zu Wasser gegeben, es wurde Eis zugesetzt, um die Temperatur auf 0-5 °C zu senken, Natriumnitritlösung wurde zugesetzt, es wurde für 4-6 Stunden bei 0-5 °C umgesetzt, als die Reaktion beendet war, wurde Aminosulfonsäure zur Entfernung der überschüssigen salpetrigen Säure zugesetzt;
b. Herstellung von p-Nitroanilin-ortho-sulfonsäure-Diazoniumsalz: p-Nitroanilin-ortho-sulfonsäure und Salzsäure wurden zu Wasser gegeben, es wurde Eis zugegeben, um auf 0-5 °C zu kühlen, Natriumnitritlösung wurde zugesetzt, es wurde bei 0-5 °C für 4-6 Stunden umgesetzt, wobei, als die Reaktion beendet war, Aminosulfonsäure zur Entfernung der überschüssigen salpetrigen Säure zugegeben wurde;
c. erster Kupplungsschritt: direktes Zugeben von H-Säure zu dem Diazoniumslz von Ansatz a, Regulieren der Temperatur auf 10-15 °C, Umsetzen für mehr als 6 Stunden, rotes Intermediat wurde erhalten;
d. zweiter Kupplungsschritt: Diazonium-Reaktionslösung von Ansatz c wurde zu dem roten Intermediat von Ansatz d gegeben, wobei Natriumcarbonat verwendet wurde, um den pH = 5-7 zu regulieren, es wurde für 2-3 Stunden umgesetzt und die Temperatur wurde auf 30-35 °C erhöht, 10-20 % des gesamten Volumens an Natriumchlorid oder Kaliumchlorid wurden zugesetzt, es wurde für 2-4 Stunden gerührt und ausgesalzt, filtriert, der Filterkuchen war das halbfertige Produkt der schwarzen Reaktivfarbstoffe;
e. Nachbehandlung: Mahlen und Lösen des halbfertigen Produktes von Ansatz d, Sprühtrocknen der Lösung, Erhalt von schwarzem Reaktivfarbstoffprodukt, orangefarbener Reaktivfarbstoffe oder rote Reaktivfarbstoffe wurden zugesetzt, es wurden schwarze "high jet"-Reaktivfarbstoffe erhalten.

## Revendications

1. Type de colorants réactifs noirs, représenté par la formule 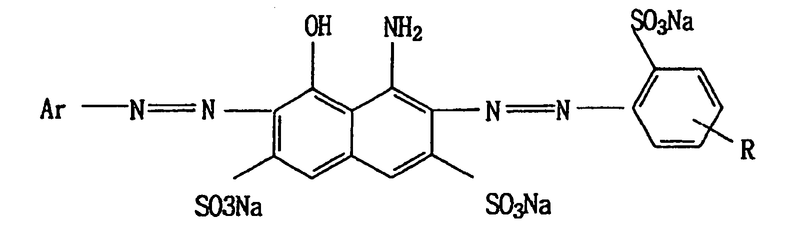 dans laquelle
R = -NHCOCHBrCH₂Br, -NHCOCBrCH₂ ou -SO₂CH=CH₂

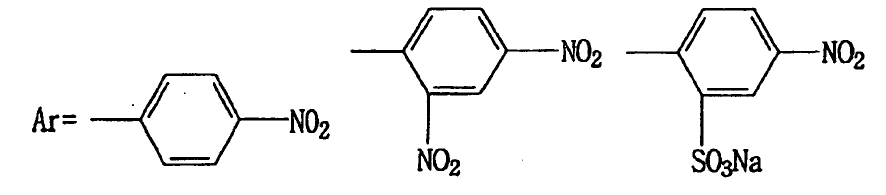

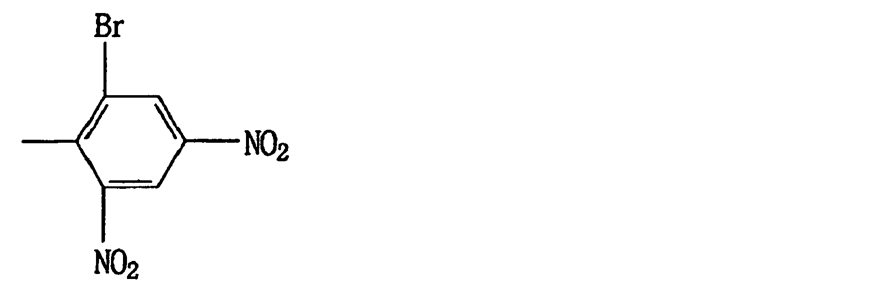

2. Procédé pour préparer des colorants réactifs noirs suivant la revendication 1, comprenant les approches suivantes :
a. préparation d'un sel de diazonium condensé : du sel de sodium d'acide 2,4-diaminobenzènesulfonique a été ajouté à de l'eau, la température a été ajustée à 2-8°C constamment en utilisant de la glace, du chlorure de 2,3-dibromopropionyle a été ajouté, le pH a été ajusté à 6-8 constamment en utilisant une solution à 10-20 % de carbonate de sodium, la réaction a été conduite pendant 3 à 6 heures, la température réactionnelle a été ajustée à 2-8°C, et la suspension du condensat a été obtenue ; de l'acide chlorhydrique et de la glace ont été ajoutés à la suspension du condensat, la suspension a été refroidie à 0-5°C, du nitrite du sodium a été ajouté, la réaction a été conduite à 7-14°C pendant 1 à 2 heures, en maintenant un léger excès de nitrite de sodium au cours du processus de réaction et, une fois la réaction parvenue à son terme, de l'acide aminosulfonique a été ajouté pour éliminer l'excès de nitrite de sodium ;
b. préparation d'un sel de diazonium de dinitroaniline : du nitrite de sodium a été ajouté à de l'acide sulfurique à 98 %, la température a été ajustée à moins de 30°C et, après l'addition a été portée à 65-70°C et maintenue pendant 2 à 3 heures ; la température a été abaissée au-dessous de 30°C, de la dinitroaniline a été ajoutée, la température a été ajustée à moins de 40°C et, après l'addition, a été maintenue à 40+3°C pendant plus de 6 heures et a été abaissée au-dessous de 30°C, le mélange a été dilué avec un mélange de glace et d'eau, la température étant inférieure à 2°C, de l'acide aminosulfonique a été ajouté pour éliminer l'excès de nitrite de sodium ;
c. préparation d'une solution d'acide H : de l'acide H quantitatif a été ajouté à de l'eau, une solution à 30-40 % d'hydroxyde de sodium a été utilisée pour la neutralisation à un pH de 6-7, ce qui a provoqué la dissolution ;
d. première étape de couplage : du bicarbonate de sodium a été ajouté au sel de diazonium de l'approche a., le pH a été ajusté à 1,5-2,5, l'acide H de cette approche a été ajouté, la température a été ajustée à 10-15°C, la réaction a été conduite pendant plus de 6 heures, ce qui a donné un intermédiaire rouge ;
e. seconde étape de couplage : la solution réactionnelle du sel de diazonium de l'approche b a été ajoutée à l'intermédiaire rouge de l'approche d, en utilisant le carbonate de sodium pour ajuster le pH à 5-7, la réaction a été conduite pendant 2 à 3 heures, la température a été portée à 30-35°C, 10-20 % du volume total de chlorure de sodium ou de chlorure de potassium ont été ajoutés, le mélange a été agité et soumis à un relargage pendant 2 à 4 heures, puis filtré, le gâteau de filtre donnant le produit semi-fini des colorants réactifs noirs ;
f. post-traitement : raffinage et dissolution du produit semi-fini de l'approche e, séchage par atomisation de la solution, obtention d'un produit noir réactif, addition de colorants réactifs oranges ou de colorants réactifs rouges, ce qui a donné les colorants réactifs noirs de premier jet.

3. Procédé pour préparer des colorants réactifs noirs suivant la revendication 1, comprenant les approches suivantes :
a. préparation d'un sel de diazonium condensé : du sel de sodium d'acide 2,4-diaminobenzènesulfonique a été ajouté à de l'eau, la température a été ajustée à 2-8°C constamment en utilisant de la glace, du chlorure de 2,3-dibromopropionyle a été ajouté, le pH a été ajusté à 6-8 constamment en utilisant une solution à 10-20 % de carbonate de sodium, la réaction a été conduite pendant 3 à 6 heures, la température réactionnelle a été ajustée à 2-8°C, et la suspension du condensat a été obtenue ; de l'acide chlorhydrique et de la glace ont été ajoutés à la suspension du condensat, la suspension a été refroidie à 0-5°C, du nitrite du sodium a été ajouté, la réaction a été conduite à 7-14°C pendant 1 à 2 heures, en maintenant un léger excès de nitrite de sodium au cours du processus de réaction et, une fois la réaction parvenue à son terme, de l'acide aminosulfonique a été ajouté pour éliminer l'excès de nitrite de sodium ;
b. préparation d'un sel de diazonium de p-nitroaniline : de l'agent tensioactif Travel Milk-12, de la p-nitroaniline et de l'acide chlorhydrique ont été ajoutés à de l'eau, un raffinage a été effectué pendant plus d'1 heure, de la glace a été ajoutée pour abaisser la température à 0-5°C, une solution de nitrite de sodium a été ajoutée, la réaction a été conduite à 0-5°C pendant 2 à 3 heures et, une fois la réaction parvenue à son terme, de l'acide aminosulfonique a été ajouté pour éliminer l'excès d'acide nitreux ;
c. préparation d'une solution d'acide H : de l'acide H a été ajouté à de l'eau, une solution d'hydroxyde de sodium à 30-40 % a été utilisée pour la neutralisation à un pH de 6-7, ce qui a provoqué la dissolution ;
d. première étape de couplage : du bicarbonate de sodium a été ajouté au sel de diazonium de l'approche a., en ajustant le pH à 1,5-2,5, l'acide H de l'approche c a été ajouté, en ajustant la température à 10-15°C, la réaction a été conduite pendant plus de 6 heures, ce qui a donné un intermédiaire rouge ;
e. seconde étape de couplage : la solution réactionnelle du sel de diazonium de l'approche b a été ajoutée à l'intermédiaire rouge de l'approche d, en utilisant le carbonate de sodium pour ajuster le pH à 5-7, la réaction a été conduite pendant 2 à 3 heures et la température a été portée à 30-35°C, 10-20 % du volume total de chlorure de sodium ou de chlorure de potassium ont été ajoutés, le mélange a été agité et soumis à un relargage pendant 2 à 4 heures, puis filtré, le gâteau de filtre donnant le produit semi-fini des colorants réactifs noirs ;
f. post-traitement : raffinage et dissolution du produit semi-fini de l'approche e, séchage par atomisation de la solution, obtention d'un produit noir réactif, addition de colorants réactifs oranges ou de colorants réactifs rouges, ce qui a donné les colorants réactifs noirs de premier jet.

4. Procédé pour préparer des colorants réactifs noirs suivant la revendication 1, comprenant les approches suivantes :
a. préparation d'un sel de diazonium condensé : du sel de sodium d'acide 2,4-diaminobenzènesulfonique a été ajouté à de l'eau, la température a été ajustée à 2-8°C constamment en utilisant de la glace, du chlorure de 2,3-dibromopropionyle a été ajouté, le pH a été ajusté à 6-8 constamment en utilisant une solution à 10-20 % de carbonate de sodium, la réaction a été conduite pendant 3 à 6 heures, la température réactionnelle a été ajustée à 2-8°C, et la suspension du condensat a été obtenue ; de l'acide chlorhydrique et de la glace ont été ajoutés à la suspension du condensat, la suspension a été refroidie à 0-5°C, du nitrite du sodium a été ajouté, la réaction a été conduite à 7-14°C pendant 1 à 2 heures, en maintenant un léger excès de nitrite de sodium au cours du processus de réaction et, une fois la réaction parvenue à son terme, de l'acide aminosulfonique a été ajouté pour éliminer l'excès de nitrite de sodium ;
b. préparation d'un sel de diazonium d'acide p-nitro-anilino-orthosulfonique : de l'acide p-nitroanilino-orthosulfonique et de l'acide chlorhydrique ont été ajoutés à de l'eau, en ajoutant de la glace pour abaisser la température à 0-5°, une solution de nitrite de sodium a été ajoutée, la réaction a été conduite à 0-5°C pendant 4 à 6 heures et, une fois la réaction parvenue à son terme, de l'acide aminosulfonique a été ajouté pour éliminer l'excès d'acide nitreux ;
c. préparation d'une solution d'acide H : de l'acide H a été ajouté à de l'eau, une solution 30-40 % d'hydroxyde de sodium à a été utilisée pour la neutralisation à un pH de 6-7, ce qui a provoqué la dissolution ;
d. première étape de couplage : du bicarbonate de sodium a été ajouté au sel de diazonium de l'approche a., en ajustant le pH à 1,5-2,5, l'acide H de l'approche c a été ajouté, en ajustant la température à 10-15°C, la réaction a été conduite pendant plus de 6 heures, ce qui a donné un intermédiaire rouge ;
e. seconde étape de couplage : la solution réactionnelle du sel de diazonium de l'approche b a été ajoutée à l'intermédiaire rouge de l'approche d, en utilisant le carbonate de sodium pour ajuster le pH à 5-7, la réaction a été conduite pendant 2 à 3 heures et la température a été portée à 30-35°C, 10-20 % du volume total de chlorure de sodium ou de chlorure de potassium ont été ajoutés, le mélange a été agité et soumis à un relargage pendant 2 à 4 heures, puis filtré, le gâteau de filtre donnant le produit semi-fini des colorants réactifs noirs ;
f. post-traitement : raffinage et dissolution du produit semi-fini de l'approche e, séchage par atomisation de la solution, obtention d'un produit noir réactif, addition de colorants réactifs oranges ou de colorants réactifs rouges, ce qui a donné les colorants réactifs noirs de premier jet.

5. Procédé pour préparer des colorants réactifs noirs suivant la revendication 1, comprenant les approches suivantes :
a. préparation d'un sel de diazonium d'ester m-sulfonaté : un ester m-sulfonaté et de l'acide chlorhydrique ont été ajoutés à de l'eau, en ajoutant de la glace pour abaisser la température à 0-5°C, une solution de nitrite de sodium a été ajoutée, la réaction a été conduite à 0-5°C pendant 4 à 6 heures et, une fois la réaction parvenue à son terme, de l'acide aminosulfonique a été ajouté pour éliminer l'excès d'acide nitreux ;
b. préparation d'un sel de diazonium de dinitroaniline : du nitrite de sodium a été ajouté à de l'acide sulfurique à 98 %, la température a été ajustée à moins de 30°C et, après l'addition a été portée à 65-70°C et maintenue pendant 2 à 3 heures ; la température a été abaissée au-dessous de 30°C, de la dinitroaniline a été ajoutée, la température a été ajustée à moins de 40°C et, après l'addition a été maintenue à 40+3°C pendant plus de 6 heures et a été abaissée au-dessous de 30°C, le mélange a été dilué avec un mélange de glace et d'eau, la température étant inférieure à 2°C, de l'acide aminosulfonique a été ajouté pour éliminer l'excès de nitrite de sodium ;
c. première étape de couplage : en ajoutant directement l'acide H au sel de diazonium de l'approche a, en ajustant la température à 10-15°C et en conduisant la réaction pendant plus de 6 heures, un intermédiaire rouge a été obtenu ;
d. seconde étape de couplage : la solution réactionnelle du sel de diazonium de l'approche c a été ajoutée à l'intermédiaire rouge de l'approche d, en utilisant le carbonate de sodium pour ajuster le pH à 5-7, la réaction a été conduite pendant 2 à 3 heures, la température a été portée à 30-35°C, 10-20 % du volume total de chlorure de sodium ou de chlorure de potassium ont été ajoutés, le mélange a été agité et soumis à un relargage pendant 2 à 4 heures, puis filtré, le gâteau de filtre donnant le produit semi-fini des colorants réactifs noirs ;
e. post-traitement : raffinage et dissolution du produit semi-fini de l'approche d, séchage par atomisation de la solution, obtention d'un produit noir réactif, addition de colorants réactifs oranges ou de colorants réactifs rouges, ce qui a donné les colorants réactifs noirs de premier jet.

6. Procédé pour préparer des colorants réactifs noirs suivant la revendication 1, comprenant les approches suivantes :
a. préparation d'un sel de diazonium d'ester m-sulfonaté : un ester m-sulfonaté et de l'acide chlorhydrique ont été ajoutés à de l'eau, en ajoutant de la glace pour abaisser la température à 0-5°C, une solution de nitrite de sodium a été ajoutée, la réaction a été conduite à 0-5°C pendant 4 à 6 heures et, une fois la réaction parvenue à son terme, de l'acide aminosulfonique a été ajouté pour éliminer l'excès d'acide nitreux ;
b. préparation d'un sel de diazonium de p-nitroaniline : de l'agent tensioactif Travel Milk-12, de la p-nitroaniline et de l'acide chlorhydrique ont été ajoutés à de l'eau, un raffinage a été effectué pendant plus d'1 heure, de la glace a été ajoutée pour abaisser la température à 0-5°C, une solution de nitrite de sodium a été ajoutée, la réaction a été conduite à 0-5°C pendant 2 à 3 heures et, une fois la réaction parvenue à son terme, de l'acide aminosulfonique a été ajouté pour éliminer l'excès d'acide nitreux ;
c. première étape de couplage : en ajoutant directement l'acide H au sel de diazonium de l'approche a, en ajustant la température à 10-15°C et en conduisant la réaction pendant plus de 6 heures, un intermédiaire rouge a été obtenu ;
d. seconde étape de couplage : la solution réactionnelle du sel de diazonium de l'approche c a été ajoutée à l'intermédiaire rouge de l'approche d, en utilisant le carbonate de sodium pour ajuster le pH à 5-7, la réaction a été conduite pendant 2 à 3 heures et la température a été portée à 30-35°C, 10-20 % du volume total de chlorure de sodium ou de chlorure de potassium ont été ajoutés, le mélange a été agité et soumis à un relargage pendant 2 à 4 heures, puis filtré, le gâteau de filtre donnant le produit semi-fini des colorants réactifs noirs ;
e. post-traitement : raffinage et dissolution du produit semi-fini de l'approche d, séchage par atomisation de la solution, obtention d'un produit noir réactif, addition de colorants réactifs oranges ou de colorants réactifs rouges, ce qui a donné les colorants réactifs noirs de premier jet.

7. Procédé pour préparer des colorants réactifs noirs suivant la revendication 1, comprenant les approches suivantes :
a. préparation d'un sel de diazonium d'ester m-sulfonaté : un ester m-sulfonaté et de l'acide chlorhydrique ont été ajoutés à de l'eau, en ajoutant de la glace pour abaisser la température à 0-5°C, une solution de nitrite de sodium a été ajoutée, la réaction a été conduite à 0-5°C pendant 4 à 6 heures et, une fois la réaction parvenue à son terme, de l'acide aminosulfonique a été ajouté pour éliminer l'excès d'acide nitreux ;
b. préparation d'un sel de diazonium d'acide p-nitro-anilino-orthosulfonique : de l'acide p-nitroanilino-orthosulfonique et de l'acide chlorhydrique ont été ajoutés à de l'eau, en ajoutant de la glace pour abaisser la température à 0-5°, une solution de nitrite de sodium a été ajoutée, la réaction a été conduite à 0-5°C pendant 4 à 6 heures et, une fois la réaction parvenue à son terme, de l'acide aminosulfonique a été ajouté pour éliminer l'excès d'acide nitreux ;
c. première étape de couplage : en ajoutant directement l'acide H au sel de diazonium de l'approche a, en ajustant la température à 10-15°C et en conduisant la réaction pendant plus de 6 heures, un intermédiaire rouge a été obtenu ;
d. seconde étape de couplage : la solution réactionnelle du sel de diazonium de l'approche c a été ajoutée à l'intermédiaire rouge de l'approche d, en utilisant le carbonate de sodium pour ajuster le pH à 5-7, la réaction a été conduite pendant 2 à 3 heures et la température a été portée à 30-35°C, 10-20 % du volume total de chlorure de sodium ou de chlorure de potassium ont été ajoutés, le mélange a été agité et soumis à un relargage pendant 2 à 4 heures, puis filtré, le gâteau de filtre donnant le produit semi-fini des colorants réactifs noirs ;
e. post-traitement : raffinage et dissolution du produit semi-fini de l'approche d, séchage par atomisation de la solution, obtention d'un produit noir réactif, addition de colorants réactifs oranges ou de colorants réactifs rouges, ce qui a donné les colorants réactifs noirs de premier jet.
